Europäisches Patentamt

(19) European Patent Office

Office européen des brevets

(11) Publication number: **0 038 567**
**B1**

(12) **EUROPEAN PATENT SPECIFICATION**

(45) Date of publication of patent specification: 23.09.87

(51) Int. Cl.⁴: **A 61 K 31/70, C 07 H 19/04**

(21) Application number: 81103043.6

(22) Date of filing: 22.04.81

(54) Deazapurine nucleoside, formulations thereof, and use thereof in therapy.

(30) Priority: 23.04.80 GB 8013411

(43) Date of publication of application:
28.10.81 Bulletin 81/43

(45) Publication of the grant of the patent:
23.09.87 Bulletin 87/39

(84) Designated Contracting States:
BE CH DE FR GB LI LU NL SE

(56) References cited:
EP-A-0 010 668
US-A-4 148 888

CHEMICAL ABSTRACTS, vol. 70, no. 11, March
16, 1969, page 377, abstract 47771n, Columbu,
Ohio, USA. Y. MIZUNO et al.: "Synthetic
studies of potential antimetabolites. XII.
Synthesis of 4-substituted 1-(B-D-
ribofuranosyl)-1H-imidazo-(4,5-c)pyridines"

(73) Proprietor: THE WELLCOME FOUNDATION
LIMITED
183-193 Euston Road
London NW1 2BP (GB)

(72) Inventor: Vinegar, Ralph
5337 Thayer Drive
Raleigh North Carolina 27609 (US)
Inventor: Wolberg, Gerald
1109 Troon Court
Cary North Carolina 27511 (US)

(74) Representative: Berg, Wilhelm, Dr. et al
Patentanwälte Dr. Berg Dipl.-Ing. Stapf Dipl.-Ing.
Schwabe Dr. Dr. Sandmair Postfach 86 02 45
Stuntzstrasse 16
D-8000 München 86 (DE)

# 0 038 567

**Description**

The present invention is concerned with a pharmaceutical formulation for topical application which is useful in medicine. More specifically it is concerned with a topical formulation useful in the treatment of inflammation by the administration of an adenosine analogue.

United States Patent No. 4 148 888 discloses 3-deazaadenosine, 4-amino-1-β-D-ribofuranosyl-1$H$-imidazo[4,5-$c$]-pyridine, as an antiviral and antifocal agent EP—A—0 010 668 discloses 3-deazaadenosine as an immunosuppressant.

3-Deazaadenosine has now been surprisingly found to possess anti-inflammatory activity and is thus useful in the treatment of inflammation and thus disease conditions associated with inflammation.

The invention accordingly provides the use of said compound or a pharmaceutically acceptable acid addition salt thereof in the manufacture of a medicament, preferably adapted for topical application, for the treatment of an inflammatory condition other than in a subject suffering from lupus erythematosis, hemolytic anemia, ulcerative colitis, nephrosis or the rejection of foreign cells including grafts and organ transplants.

By the term 'inflammation' is meant the reactive state of hyperaemia and exudation from its blood vessels, with consequent redness, heat, swelling and pain, which a tissue enters in response to physical or chemical injury or bacterial invasions.

Clinical conditions with which inflammation is associated, and hence for which an anti-inflammatory agent is indicated, include for example arthritis, including rheumatoid arthritis and osteoarthritis, post operative inflammation, dental inflammation, acute and chronic ocular inflammatory diseases, conjunctivitis.

3-Deazaadenosine contained in a pharmaceutical formulation adapted for topical application has been found to be a potent, long-acting anti-inflammatory agent. 3-Deazaadenosine is useful in the treatment of both acute and chronic inflammation. 3-Deazaadenosine has been found to inhibit both developing and established adjuvant arthritis, an animal model in the rat of human rheumatoid arthritis, and is thus particularly useful in the treatment of acute and chronic rheumatoid arthritis.

The mechanism of the anti-inflammatory action of 3-deazaadenosine is at present unknown. It is not antipyretic and has no analgesic activity *per se,* and in this respect is like steroidal anti-inflammatory drugs such as prednisolone and hydrocortisone and like these known drugs will give relief from pain in many clinical syndromes by reducing inflammation. 3-Deazaadenosine does not inhibit prostaglandin metabolism *in vitro* through either the lipoxygenase or cyclooxygenase pathway and is thus free of the side effects, particularly gastric damage and inhibition of platelet aggregation, associated with prostaglandin inhibitors such as acetylsalicylic acid. 3-Deazaadenosine is also free of the side effects of the anti-inflammatory steroids and unlike compounds such as acetaminophen (paracetamol) does not cause liver damage in the animal models used.

Although its mechanism of action is different to that of acetylsalicylic acid, 3-deazaadenosine is similar to acetylsalicylic acid in that it has a long duration of action (about 15 hours) associated with a short half life. Thus 3-deazaadenosine will require relatively infrequent administration for example twice daily and the problem of morning stiffness and associated pain and crippling effects in patients with arthritic conditions may be alleviated, in contrast to the shorter acting non-steroidal and steroidal anti-inflammatory agents which do not possess this property. In addition 3-deazaadenosine, by virtue of its different mode of action to acetyl salicylic acid, salicylates and steroids, may be combined with such drugs to provide a 'sparing effect' reducing the required dose of these drugs and hence the side effects associated therewith. 3-Deazaadenosine is also advantageous in possessing a high therapeutic index and thus is unlikely to present problems associated with accidental overdose. Its high water solubility is a yet further advantage.

The amount of 3-deazaadenosine required to reduce inflammation is relatively low and lies in the range of 0.1 to 30 mg/kg per day, particularly 0.2 to 5 mg/kg per day, preferably 0.3 to 1 mg/kg per day.

In contrast immunesuppression requires more than 30 mg/kg and antiviral activity requires 100—200 mg/kg per day. Thus effects associated with other activities of 3-deazaadenosine, in particular suppression of the immune response, are expected to be absent or minimal at the dose range required for anti-inflammatory activity.

As 3-deazaadenosine is effective for both chronic and acute, i.e. both long and short term, inflammation, the compound may be administered for as long as the inflammation remains, which may be for several hours to a number of years.

When 3-deazaadenosine is administered in the form of a pharmaceutically acceptable salt then the salt will be non-toxic salt, suitably an acid addition salt. Suitable salts include those derived from hydrochloric acid, hydroiodic acid, sulphuric acid, phosphoric acid, acetic acid, p-toluene sulphonic acid, methane sulphonic acid, maleic acid, lactic acid, citric acid, tartaric acid, succinic acid, oxalic acid, p-chlorobenzenesulphonic acid, isethionic acid, glucuronic acid, pantothenic acid, and lactobionic acid.

While it is possible for 3-deazaadenosine or a pharmaceutically acceptable salt thereof (hereinafter referred to as "the active compounds") to be administered as the raw chemical it is preferably presented in the form of a pharmaceutical formulation.

A pharmaceutical formulation according to the present invention will comprise an active compound together with a pharmaceutically acceptable carrier therefor. The carrier must be "acceptable" in the sense

2

of being compatible with the other ingredients of the formulation and not deleterious to the recipient thereof. The pharmaceutical formulation may be prepared by any of the methods well known in the art of pharmacy all of which involve bringing the active compound into association with the carrier therefor.

3-Deazaadenosine is effective as an anti-inflammatory agent not only orally and systemically but also locally and is thus particularly suitable for topical administration. The term "topical" as applied herein relates to the use of the active ingredient incorporated in a suitable pharmaceutical carrier, and applied at the site of the disease for exertion of local action. Included within the scope of topical formulations are ophthalmic formulations.

Pharmaceutical formulation suitable for topical administration may be presented in anhydrous forms such as lotions, jellies, sprays, aerosols, bath oils or preferably ointments. The term ointment includes formulations (including creams) having oleaginous, absorption, water-soluble and emulsion type bases, for example petrolatum, lanolin, polyethylene glycols and mixtures thereof. Ointments are semi-solid materials with the active compound dispersed therein. These and other topical formulations enable the active ingredient to be applied and retained locally at the site of the disease.

Topical formulation may contain a concentration of the active compound of from 0.01 to 10% w/w preferably 0.1 to 1% w/w most preferably 0.2 to 0.5% w/w. Topical formulations may be applied locally one or more times daily, as required.

The invention also provides a pharmaceutical formulation adapted for only topical administration to the exclusion of oral and injectable administration comprising as the active ingredient 3-deazaadenosine or a pharmaceutically acceptable addition salt thereof together with a pharmaceutically acceptable carrier therefor.

All methods for the preparation of such formulations include the step of bringing into association the active compound with liquid carriers or finely divided solid carriers or both and then, if necessary, shaping, the products into the desired formulation.

It should be understood that in addition to the aforementioned carrier ingredients the pharmaceutical formulations described above may include, as appropriate, one or more additional carrier ingredients such as diluents, buffers flavouring agents, binders, surface active agents, thickeners, lubricants and preservatives (including anti-oxidants), and substances included for the purpose of rendering the formulation isotonic with the blood of the intended recipient.

The invention will now be illustrated by the following Examples which should in no way be considered as a limitation thereof.

Example 1
Acute anti-inflammatory activity of 3-deazaadenosine: carrageenan pleurisy assay (CPA)

Following the procedure previously described by Vinegar et al. in *Proc. Soc. Exp. Biol. Med., 143, 711,* (1973) as recently modified/Vinegar et al., *Eur. J. Rheum. Inflamm., 1,* 204 (1978)/the acute anti-inflammatory activity of 3-deazaadenosine was compared with that of known anti-inflammatory drugs. The average 3 hour exudate volume for each drug treated group was determined and the % inhibition relative to solvent-fed control animals calculated, the $ED_{50}$ (mg/kg body weight) being the dose required to reduce the 3 hour exudate volume by 50%. There were 5 animals in each drug-treated group and the control group. The results are shown in Table 1:—

TABLE 1
$ED_{50}$ (mg/kg)

| | 3-Deazaadenosine | Acetylsalicylic acid | Prednisolone | Hydrocortisone |
|---|---|---|---|---|
| 3 hr. vol. p.o. | 2.8±0.65 | 28±3.2 | 3±0.50 | 14±10.2 |

Example 2
Duration of action of anti-inflammatory activity of 3-deazaadenosine

By means of the Carrageenan Pleurisy Assay of Example 1 the duration of action of the tests compounds after a single oral pretreating dose was determined. For 3-deazaadenosine the effect lasted for 14.9 hours at 6 mg/kg (p.o.) whilst for acetylsalicylic acid (150 mg/kg p.o.) and acetaminophen (220 mg/kg p.o.) the effect lasted for 35 hours and 2 hours respectively. This value represents the time (hours) of drug administration prior to the injection of carrageenan in which the inhibition of the 3 hour pleural exudate volume declined to 40%.

Example 3
Local anti-inflammatory activity of 3-deazaadenosine: carrageenan pleurisy assay (CPA)

Following the procedure described by Vinegar et al./*Eur. J. Rheum. Inflamm., 1,* 204, (1978)/the local anti-inflammatory activity of 3-deazaadenosine was determined and compared to that of standard

anti-inflammatory drugs. There were 5 animals in each drug-treated group and the control group. The results are shown in Table 2:—

TABLE 2
$ED_{50}$ (mg/kg)

|  | 3-Deazaadenosine | Hydrocortisone | Acetylsalicylic acid |
|---|---|---|---|
| 3 hr. vol. local (intra-pleural) | 0.05 | 0.1 | 0.04 |

Example 4

Effect of 3-deazaadenosine against adjuvant arthritis

The effect of 3-deazaadenosine in rats against both developing and established adjuvant arthritis was determined by the method described by R. Vinegar *et al. Journal of Immunopharmacology. 1,* 483, (1979). The scoring procedure of A. L. F. Currey and M. Ziff, *J. Exp. Med., 121,* 185 (1968) was used to assess the arthritic joint scores of rats. There were 5 animals in each drug-treated group. For developing adjuvant arthritis the drugs were administered in the diet from day 1 to day 16. For established adjuvant arthritis the drugs were administered from day 21 to 42. The results are shown in Table 3 below.

TABLE 3

| Drug | Developing arthritis $ED_{50}$ on day 16 mg/kg/day in diet. | Established arthritis $ED_{25}$ on day 42 mg/kg/day in diet. |
|---|---|---|
| 3-deazaadenosine | 8±2.8 | (18)* |
| Acetylsalicylic acid | 150±53.1 | 150 |
| Prednisolone | 1±0.7 | 0.2 |

* Parentheses indicate an approximate value.

Example 5

| Water soluble ointment | Amount (g) |
|---|---|
| 3-Deazaadenosine | 0.5 |
| Polyethylene glycol 300 | 20.0 |
| Polyethylene glycol 1500 | 79.5 |
| | 100.0 |

Example 6

| Skin cream | Amount (g) |
|---|---|
| 3-Deazaadenosine | 0.5 |
| Glyceryl monostearate | 20.0 |
| Methylparben | 0.3 |
| Petrolatum light liquid | 4.0 |
| Propylene glycol | 5.0 |
| Span® 60 | 2.0 |
| Tween® 61 | 4.0 |
| Water | 64.6 |
| Total | 100.0 |

**Claims**

1. A pharmaceutical formulation adapted for only topical application to the exclusion of oral and

4

injectable administration and comprising, as the active ingredient, 4-amino-β-*D*-ribofuranosyl-1H-imidazo-[4,5-c]-pyridine or a pharmaceutically acceptable acid addition salt thereof together with a pharmaceutically acceptable carrier therefor.

2. A formulation as claimed in claim 1 in the form of a lotion, jelly, spray, aerosol, bath oil, cream or ointment.

3. A formulation as claimed in claim 2 in which the ointment has an oleaginous, absorption, water-soluble or emulsion-type base.

4. A formulation as claimed in either of claims 2 or claim 3 in which the base comprises petrolatum, lanolin, polyethylene glycols and mixtures thereof.

5. A formulation as claimed in either of claim 3 or claim 4 in the form of a cream.

6. A formulation as claimed in any one of claims 1 to 3 in which the concentration of the active ingredient is in the range of from 0.01% to 10% w/w.

7. The use of 4-amino-β-*D*-ribofuranosyl-1H-imidazo-[4,5-c]-pyridine or a pharmaceutically acceptable acid addition salt thereof in the manufacture of a medicament for the treatment of an inflammatory condition other than in a subject suffering from lupus erythematosis, hemolytic anemia, ulcerative colitis, nephrosis or the rejection of foreign cells including grafts and organ transplants.

8. The use according to claim 7 wherein the medicament is for administration of from 0.1 to 30 mg of said 4-amino-β-*D*-ribofuranosyl-1H-imidazo-[4,5-c]-pyridine or pharmaceutically acceptable acid addition salt thereof, per kilogram of recipient bodyweight per day.

9. Use as claimed in claim 7 or claim 8, wherein the inflammatory condition is selected from arthritis, post-operative inflammation, dental inflammation, acute and chronic inflammatory diseases and conjunctivitis.

10. Use as claimed in any of claims 7—9 wherein the medicament is for treatment by topical application.

## Patentansprüche

1. Pharmazeutische Formulierung, adaptiert für eine ausschließlich topische Applikation, wobei orale und injizierbare Verabreichungsformen ausgeschlossen sind, enthaltend als aktiven Bestandteil 4-Amino-β-D-ribofuranosyl-1H-imidazo-[4,5-c]-pyridin oder ein pharmazeutisch verträgliches Säureadditionssalz zusammen mit einem dafür verträglichen Trägerstoff.

2. Pharmazeutische Formulierung nach Anspruch 1 in Form einer Lotion, eines Gelees, eines Sprays, eines Aerosols, eines Badeöls, einer Creme oder eine Salbe.

3. Pharmazeutische Formulierung nach Anspruch 2, in der die Salbe eine ölhaltige, die Absorption ermöglichende, wasserlösliche oder emulsionsartige Grundlage besitzt.

4. Pharmazeutische Formulierung nach Anspruch 2 oder 3, in der die Grundlage Petrolatum, Lanolin, Polyethylenglykole und Mischungen davon enthält.

5. Pharmazeutische Formulierung nach einem der Ansprüche 3 oder 4 in Form einer Creme.

6. Pharmazeutische Formulierung nach einem der Ansprüche 1 bis 3, in der die Konzentration des aktiven Bestandteiles im Bereich von 0,01% bis 10% w/w liegt.

7. Verwendung von 4-Amino-β-D-ribofuranosyl-1H-imidazo-[4,5-c]-pyridin oder eines pharmazeutisch verträglichen Säureadditionssalzes zur Herstellung eines Medikaments zur Behandlung eines entzündlichen Zustandes, verschieden von dem Zustand eines Subjektes, das an Lupus erythematosis, haemolytischer Anaemie, ulcerativer Colitis, Nephrosis oder an der Abstoßung von fremden Zellen einschließlich von Transplantaten und Organtransplantaten, leidet.

8. Verwendung nach Anspruch 7, bei der das Medikament zur Verabreichung von 0,1 bis 30 mg 4-Amino-β-D-ribofuranosyl-1H-imidazo-[4,5-c]-pyridin oder eines pharmazeutisch verträglichen Säureadditionssalzes pro Kilogramm Körpergewicht des Empfängers pro Tag anzuwenden ist.

9. Verwendung nach Anspruch 7 oder 8, bei der der entzündliche Zustand einem der folgenden Zustände, nämlich Arthritis, postoperativer Entzündung, Zahnentzündung, akuten und chronisch entzündlichen Krankheiten und Konjunktivitis entspricht.

10. Verwendung nach einem der Ansprüche 7 bis 9, bei der das Medikament für eine Behandlung durch topische Applikation anzuwenden ist.

## Revendications

1. Composition pharmaceutique propre à la seule application par voie topique à l'exclusion de l'administration par voie orale et par injection et comprenant comme constituant actif de la 4-amino-β-*D*-ribofuranosyl-1H-imidazo-[4,5-c]-pyridine ou un sel d'addition d'acide pharmaceutiquement acceptable de celle-ci, conjointement avec un excipient pharmaceutiquement acceptable.

2. Composition suivant la revendication 1, sous la forme d'une lotion, d'une gelée, d'une pulvérisation, d'un aérosol, d'une huile pour le bain, d'une crème ou d'un onguent.

3. Composition suivant la revendication 2, dans laquelle l'onguent a une base de type oléagineux, d'absorption, hydrosoluble ou en émulsion.

4. Composition suivant la revendication 2 ou 3, dans laquelle la base comprend de la vaseline, de la lanoline, des polyéthylèneglycols et leurs mélanges.

5. Composition suivant l'une quelconque des revendications 3 ou 4, sous la forme d'une crème.

6. Composition suivant l'une quelconque des revendications 1 à 3, dans laquelle la concentration du constituant actif se situe dans l'intervalle de 0,01 à 10% p/p.

7. Utilisation de la 4-amino-β-*D*-ribofuranosyl-1H-imidazo-[4,5-c]-pyridine ou d'un sel d'addition d'acide pharmaceutiquement acceptable de celle-ci pour la préparation d'un médicament pour le traitement d'un état inflammatoire autre que chez un sujet souffrant de lupus érythémateux, d'anémie hémolytique, de colite ulcéreuse, de néphrose ou de rejet de cellules étrangères, y compris les greffes et organes transplantés.

8. Utilisation suivant la revendication 7, dans laquelle le médicament est destiné à l'administration de 0,1 à 30 mg de la 4-amino-β-*D*-ribofuranosyl-1H-imidazo-[4,5-c]-pyridine ou d'un sel d'addition d'acide pharmaceutiquement acceptable de celle-ci par kg de poids du corps du receveur et par jour.

9. Utilisation suivant la revendication 7 ou 8, dans laquelle l'état inflammatoire est choisi parmi l'arthrite, l'inflammation post-opératoire, l'inflammation dentaire, les affections inflammatoires aiquës et chroniques et la conjonctivite.

10. Utilisation suivant l'une quelconque des revendications 7 à 9, dans laquelle le médicament est destiné au traitement par application topique.